# EUROPEAN PATENT SPECIFICATION

(11) **EP 0 366 043 B1**
(45) Date of publication and mention of the grant of the patent: **30.03.1994**
(21) Application number: 89119648.7
(22) Date of filing: 23.10.1989
(51) Int. Cl.: C12P 21/08, A61K 39/395, G01N 33/577, G01N 33/68, C07K 3/18

(54) **Monoclonal antibody**
Monoklonaler Antikörper
Anticorps monoclonal

(30) Priority: 24.10.1988 JP 267897/88
(43) Date of publication of application: 02.05.1990
(73) Proprietor: OTSUKA PHARMACEUTICAL CO., LTD., Chiyoda-ku Tokyo (JP)
(72) Inventor: Ohmoto, Yasukazu, Itano-gun Tokushima (JP); Nishida, Tsutomu, Naruto-shi Tokushima (JP); Mizuno, Keiko, Tokushima-shi Tokushima (JP); Nakai, Satoru, Itano-gun Tokushima (JP)
(74) Representative: Wächtershäuser, Günter, Prof. Dr.

(56) References cited:
- Hybridoma, Vol. 6, No. 4, p. 359-370, 1987 B.M. Fendly et al., "Murinemonoclonal Antibodies defining neutralizing epitopes on Tumor Necrosis Factor"
- Hybridoma, Vol. 6, No 5, p. 489-507, 1987 T.S. Bringman et al., "Monoclonalantibodies to human Tumor Necrosis Factors alpha and beta: Application foraffinity purification, immunoassays, and as structural probes"

## Description

### BACKGROUND OF THE INVENTION

Field of the Invention:

The present invention relates to monoclonal antibodies for human TNFs (tumor necrosis factors), and, more particularly, to monoclonal antibodies for a human TNF which are useful as a medicine and ensure immunological purification and measurement of said human TNF.

### Description of the Background Art:

A lipopolysaccharide (LPS), when administered to a mouse, rabbit, or rat sensitized by *Bacillus de Calmette Guerin* (BCG), induces a factor hemorrhagically necrosing tumors in serum. *Carswell et al.* named this factor "TNF" in 1975 [*Proc. Nat. Acad. Sci.,* USA, *72.* 3666 (1975)]. Macrophages proliferate in the spleen of a mouse sensitized with BCG but elapse by the administration of LPS. From this, TNF has been considered to be produced by macrophages. Recent studies have unveiled the fact that an *in vitro* LPS treatment of isolated macrophages induced a TNF activity in the supernatant of the macrophage culture broth, confirming that macrophages are TNF-producing cells. Several leukemia cells producing TNF have also been reported.

The characteristic of TNF destroying various cancer cells and inhibiting their growth, while exhibiting no such effects on normal cells, suggests its potential utility as a carcinostatic agent. The anti-tumor activity of TNF has been confirmed in experiments using a purified TNF derived from mice and rabbits.

The TNF activity has been measured using a fibroblast L929 having a strong sensitivity to a TNF. The TNF titer is generally expressed by its concentration which can collapse 50% of L929 cells cultured on a culture plate as one unit.

The molecular weights of TNF produced by various animals as determined by a gel filtration analysis are reported to be 150,000 and 40,000-60,000 for TNF produced by mouse, 67,000 and 39,000 for TNF produced by rabbit, and 34,000-140,000 for TNF produced by human. According to molecular weight determination using SDS-PAGE, purified TNFs derived from rabbit and human are reported to be 17,000. TNFs having this molecular weight are termed "TNF-α". Natural TNFs are considered to exist as oligomers.

In 1984, the cloning of TNF-CDNA was successfully achieved using human myeloma leukemia cells "HL-60" which are a type of TNF-producing cell. This has ensured large-scale production of TNF using *E. coli.* The cDNA cloning revealed the fact that human TNF is constituted by 157 amino acids and has a long preceding polypeptide having 76 amino acids [D. Pennica et al., *Nature, 312,* 724-729 (1984)]. Very closely following this, the successful cloning of chromosomal genes was also reported [T. Shirai et al., *Nature, 313,* 803-806 (1985)], and it was revealed that human TNF genes are composed of four exons.

The amino acid sequence of TNF and the gene base sequence have respectively 28% and 46% homologies with a cytotoxin factor and a lymphotoxin (LT) produced by B-cells. TNF, however, has a substantive difference from the LT in that the former has no N-glycosil-type sugar chain bonding site. Although TNF and LT have no immunological cross-reactivity, they exhibit very similar cytotoxicity. The homological portions of TNF and LT are considered to be involved in the cytotoxicity.

As mentioned above, because of its special physiological activity, the utility of TNF as a medicine can be expected. A number of studies have been undertaken with a view to utilize TNF as a medicine. Also, extensive researches are underway on the subject of various immunodeficiencies and immunoreactions, as well as on the subject of TNF assay in clinical samples of diseases related to immunodeficiencies and immunoreactions.

A bioassay technique is currently used for the analysis of the aforementioned TNF-α. According to this method TNF is measured in terms of its activity. The method, however, has problems in its complicated procedure and insufficient accuracy. The method also requires stringent precaution concerning any components interfering with the assay result. Development of a measurement technique substituting for the bioassay technique has therefore been sought.

### SUMMARY OF THE INVENTION

Accordingly, an object of the present invention is to provide monoclonal antibodies exhibiting specific reactivities to human TNF-α which are useful as a medicine and ensure immunological purification and measurement of said human TNF.

Other objects, features and advantages of the invention will hereinafter become more readily apparent from the following description.

### DETAILED DESCRIPTION OF THE INVENTION AND PREFERRED EMBODIMENTS

According to the present invention monoclonal antibodies having a specificity for human TNF-α is provided. The monoclonal antibodies are obtainable from the hybridoma KOCO 705 (FERM BP-2569).

A novel and simple immunoassay technique for measuring said human TNF-α with high sensitivity and with high precision can be provided by the use of the monoclonal antibodies of the present invention.

Since the monoclonal antibodies of the present invention are specific with respect to TNF-α, they can be utilized for a specific purification of TNF-α if applied to affinity chromatography or the like.

The antibodies of the present invention are hereinafter described in detail.

The antibodies of the present invention can be prepared by using a TNF-α as an immune antigen. More specifically, a hybridoma is first prepared from mammal phlogocytes (immunocytes) immuned with said immune antigen and mammal plasmacytoma cells. A clone capable of producing a desired antibody is selected from the hybridoma, cultured, and collected.

There are no specific limitations as to the types of TNF-α used as an immune antigen in this method. A supernatant of a culture medium containing a human TNF induced by the application of a known *in vitro* method or its purified product [*Proc. Natl. Acad. Sci.,* USA, *82**,* 6637 (1985)], a human TNF produced by the use of a gene recombinant technique [*Nature, 312,* 724-729 (1984)], or a synthetic peptide prepared having a part of the amino acid sequence of one of these TNFs can be used.

There are no specific limitations as to mammals to be immuned with the immune antigen. It is desirable, however, that the mammal be selected taking into consideration its compatibility with plasmacytoma cells used for the cell fusion. Usually, the use of mouse or rat is advantageous.

Immunization can be performed by administering said immune antigen to a mammal by intravenous, subcutaneous, intradermic, or intraperitoneal injection. More specifically, the immune antigen, if desired, mixed with a conventional adjuvant, is administered to an animal, e.g. mouse, once a day at 3-5 intervals until the total amount administered becomes about 100-500 µg/mouse. Preferable immune antigens are spleen cells extracted about 3 days after the above final administration.

There are several cells known in the art as plasmacytoma cells of mammals which can be used as the parent cells to be fused with the immune cells. They are, for example, myeloma tumor cells such as p3 (p3/x63-Ag8) [*Nature, 256,* 495-497 (1975)], p3-U1 [*Current Topics in Microbiology and Immunology, 81,* 1-7 (1978)], NS-1 [*Eur. I. Immunol., 6,* 511-519 (1976)], MPC-11 [*Cell, 8,* 405-415 (1976)], SP2/O [*Nature, 276,* 269-270 (1978)], FO [*J. Immunol. Meth., 35,* 1-21 (9180)], x63.6.5.3. [*J. Immunol., 123,* 1548-1550 (9179)], S194 [*J. Exp. Med., 148,* 313-323 (1978)], etc., rat R210 [*Nature, 277,* 131-133 (1979)], and the like.

Fusion of these plasmacytoma cells and the above immune cells can be carried out according to a known method, for example, according to the method of Milstein [*Method in Enzymology, 73,* 3 (1981)]. Specifically, the cell fusion can be carried out in a normal culture medium in the presence of a conventional cell fusion promoter such as polyethylene glycol (PEG), *Sendai virus* (HVJ), etc. An auxiliary agent such as dimethylsulfoxide may be added to the culture medium, if necessary for further promoting the cell fusion efficiency. The ratio of plasmacytoma cells and immune cells used may be a ratio commonly employed in the art. Usually, immune cells are used in an amount of 1-10 times plasmacytoma cells. Various media commonly used for culturing plasmacytoma cells, RPMI-1640 medium, MEM medium, etc., can be used for the cell fusion. It is desirable that serum replenishers such as fetus cattle serum (FCS) be eliminated from the medium. The cell fusion is carried out by thoroughly mixing a prescribed amount of plasmacytoma cells and immune cells in a medium and by adding to it about 30-60 w/v% of a PEG solution having an average molecular weight of 1,000-6,000 which is heated in advance to about 37°C. Thereafter, the addition of a suitable medium and centrifugation to remove the supernatant are repeated until a desired hybridoma is formed.

Separation of the hybridoma can be carried out by culturing the hybridoma in a conventional selection medium, such as, for example, HAT medium (a medium containing hypoxanthine, aminopterin, and thymidine). The culture is carried out for a period of time, usually several days to several weeks, sufficient to destroy cells other than target hybridoma such as unfused cells and the like. The hybridoma thus produced is subjected to screening of the target antibody by means of a conventional limiting dilution analysis and to monocloning.

The screening of the target antibody can be carried out by any one of various methods commonly used for antibody screening, such as the ELISA method [Engvall, E., *Meth. Enzymol., 70,* 419-439 (9180)], the plaque method, the spot method, the agglutination reaction method, the Ouchterlony method, the radio-immunoassay (RIA) method, and the like ["Hybridoma and Monoclonal Antibody", R&D Planning Publishing Co., 30-53 (1982)]. The above-mentioned immune antigen can be used for the screening.

The hybridoma thus obtained capable of producing desired monoclonal antibodies which can identify human TNF-α can be subcultured in a normal medium and can be stored in liquid nitrogen for a long period of time.

Collection of desired antibodies from the hybridoma can be performed, for example, by culturing the hybridoma according to a conventional method and collecting the supernatant containing antibodies, or by administering the hybridoma to a mammal having compatibility therewith, allowing the hybridoma to grow, and collecting the antibodies from the abdominal dropsy. The former method is suitable for preparing a high-purity antibody and the latter can be applied to a large-scale production of antibodies.

The antibodies thus produced can be purified by a conventional method such as salting-out, gel permeation, affinity chromatography, or the like.

The monoclonal antibodies of the present invention thus prepared have a specificity for TNF-α.

The antibodies of the present invention have a neutralizing effect on human TNF-α biological activity. This antibody is particularly suitable for the specific measurement of human TNF-α having a biological activity. Also, among the antibodies, described here, there is one of a type which is capable of recognizing specifically different sites of TNF-α, exhibits no steric hindrance between themselves, and, at the same time, can combine with TNF-α. Such an antibody is effective as a sample for the sandwich technique and the like. In addition, the antibodies of the present invention exhibit a particularly superior reactivity in liquid-phase or solid-phase systems. This antibody is suitable for use in liquid- or solid-phase immunoassay.

According to the present invention monoclonal antibodies which have a specificity to human TNF-α are provided. Also, an immunoassay technique having extremely high sensitivity and superior specificity can be provided by the use of antibodies of the present invention. The immunoassay technique can detect and measure with a very high precision human TNF-α in samples such as clinical samples containing even an extremely small amount of human TNF-α.

Other features of the invention will become apparent in the course of the following description of the exemplary embodiments which are given for illustration of the invention and are not intended to be limiting thereof.

### EXAMPLES

### Example 1

### Preparation of Antibodies of the Present Invention

A human TNF-α (10-20 µg) prepared by a gene recombinant technique [*Nature, 312,* 724-729 (1984)] was intraperitoneally administered to a BALB/c mouse together with a complete Freund's adjuvant. The same amount of the human TNF-α was additionally administered twice together with an incomplete Freund's adjuvant, first 3-4 weeks after the initial administration and second 3-4 weeks thereafter, thus immunizing the mouse. After 3-4 days following the last administration, cell fusion was performed according to a conventional method [*Method in Enzymology, 73,* 3 (1981)], using immunized spleen cells and myeloma tumor cells [P3U1, *Current Topics in Microbiology, 81,* 1-7 (1978)] at a ratio of 10:1 in polyethylene glycol (PEG-4000). The hybridoma was screened with the HAT medium. The supernatant was tested by an enzymatic immunoassay technique using a 96-well microplate coated with said human TNF-α and a (goat) peroxidase-labeled anti-mouse IgG (product of E. Y. Laboratories, Inc.), thereby detecting cells capable of producing the target antibody for the human TNF-α.

Cloning by a limiting dilution technique was repeated to produce 7 strains of clone capable of producing the desired antibodies. They were named KOCO701, KOCO702, KOCO703, KOCO704, KOCO705, KOCO706, and KOCO707. The antibodies produced by them were respectively named ANOC701, ANOC702, ANOC703, ANOC704, ANOC705, ANOC706, and ANOC707.

One of the strains, the hybridoma producing an antibody with the present invention, "KOCO 705", was deposited to the Fermentation Research Institute under the name of KOCO 705 (FERM BP-2569).

Characteristics of the antibodies produced by each of the above clones are as follows:

### (1) Antibody Subclass

The subclasses were determined by using a mouse antibody subclass detection kit (product of Bio-Rad Co.). The results are given in Table 1.

**TABLE 1**

| Antibody No. | Subclass |
|---|---|
| ANOC701 | IgG₁ |
| ANOC702 | IgG₁ |
| ANOC703 | IgG₂ₐ |
| ANOC704 | IgG₁ |
| ANOC705 | IgG₁ |
| ANOC706 | IgG₁ |
| ANOC707 | IgG₁ |

### (2) Antibody Production Level

IgG in ascites was purified using the Protein A affinity, and the concentration (mg/ml) was determined by OD₂₈₀ adsorption. The OD₂₈₀ value at a 1 mg/ml IgG concentration was assumed to be 1.4.

The results are shown in Table 2.

**TABLE 2**

| Antibody No. | IgG (mg/ml) |
|---|---|
| ANOC701 | 0.1 |
| ANOC702 | 1.6 |
| ANOC703 | 0.2 |
| ANOC704 | 1.2 |
| ANOC705 | 1.1 |
| ANOC706 | 8.6 |
| ANOC707 | 2.0 |

### (3) Neutralization Antibody Titer

The neutralization antibody titer of the ascites was determined by the LM assay technique which is a type of TNF bioassay. The neutralization antibody titer is defined as the unit numbers of a TNF-α which can be neutralized by 1 ml of an ascites to one unit.

The results are shown in Table 3.

**TABLE 3**

| Antibody No. | Neutralization Antibody Titer (U) |
|---|---|
| ANOC701 | 32,000 < |
| ANOC702 | 32,000 < |
| ANOC703 | 2,000 > |
| ANOC704 | 32,000 < |
| ANOC705 | 32,000 < |
| ANOC706 | 32,000 < |
| ANOC707 | 32,000 < |

### (4) Molecular Weight

Hybridoma cultured in the abdomen of a mouse was purified into IgG₁ using an IgG purification kit (MOPS Kit, product of Bio-rad Co.). The IgG's heavy-chain and light-chain molecular weights were determined by electrophoresis using SDS-PAGE in the presence of 2ME. The sum of the two molecular weights was taken as the monoclonal antibodies.

The results are shown in Table 4.

**TABLE 4**

| Antibody No. | Molecular Weight | | |
|---|---|---|---|
| | Heavy chain | Light chain | IgG |
| ANOC701 | N.T. | N.T. | N.T. |
| ANOC702 | 53.0 | 25.0 | 156 |
| ANOC703 | 53.0 | 24.5 | 155 |
| ANOC704 | 52.0 | 24.0 | 152 |
| ANOC705 | 53.0 | 24.5 | 155 |
| ANOC706 | 53.0 | 23.0 | 152 |
| ANOC707 | 50.0 | 24.0 | 148 |

### (5) Western Blotting Analysis

Specificity for TNF-α and non-reactivity with *E. coli.* of the antibodies of the present invention were confirmed in the following manner. τTNF-α and τTNF-α-producing *E. coli* were treated with an SDS lysis buffer to a final concentration of 100 µg/ml. SDS-PAGE was performed to effect blotting onto nitrocellulose and to determine the reactivity of each monoclonal antibody.

The results are shown in Table 5.

**TABLE 5**

| Antibody No. | Reactivity | |
|---|---|---|
| | *E. coil.* protein | TNF-α |
| ANOC701 | - | + + |
| ANOC702 | - | + + + + |
| ANOC703 | - | - |
| ANOC704 | - | + + + + |
| ANOC705 | - | + + |
| ANOC706 | - | + + |
| ANOC707 | - | + + |

Table 5 shows that ANOC701, ANOC702, ANOC704, ANOC705, ANOC706, and ANOC707 combine with TNF-α with specificity and do not react with *E. coli* protein. The non-reactivity of ANOC703 with τTNF-α suggests its recognition of a steric structure.

### (6) ELISA Sensitivity

Each 100 µl of monoclonal antibodies ANOC701-ANOC707 diluted with PBS to a concentration of 10 µg/ml was poured into each well of a 96-well microplate and left overnight. Holes were blocked with 1% skim milk. 100 µl of a standard TNF-α was added to each well and incubated at 4°C overnight. After washing the plate, 100 µl of a 1,000-fold solution of a rabbit polyclonal antibody (OCT701) against TNF-α was added and incubated for 2 hours at room temperature. The plate was washed and 100 µl of (goat) POD-labeled anti-rabbit IgG was added to each well. After incubation for 2 hours at room temperature, the plate was washed and the enzymatic activity of POD was measured.

The ELISA sensitivity of a monoclonal antibody is expressed by the TNF-α concentration of the antibody, the difference of which OD₄₉₂ adsorption and the OD₄₉₂ adsorption without TNF-α is 0.1.

The results are shown in Table 6.

**TABLE 6**

| Antibody No. | ELISA Sensitivity (OD₄₉₂=0.1) |
|---|---|
| ANOC701 | N.T. |
| ANOC702 | 1.0 ng/ml (0.10 ng/well) |
| ANOC703 | N.T. |
| ANOC704 | 1.2 ng/ml (0.12 ng/well) |
| ANOC705 | 0.44 ng/ml (0.044 ng/well) |
| ANOC706 | 0.86 ng/ml (0.086 ng/well) |
| ANOC707 | 1.0 ng/ml (0.10 ng/well) |

### (8) Standard Curve by ELISA Method

100 µl of monoclonal antibody ANOC705 diluted with PBS to a concentration of 10 µg/ml was poured into each well of a 96-well microplate and left overnight at 4°C. Wells were blocked with 1% skim milk. 100 µl of a standard TNF-α was added in triplicate to each well and incubated at 4°C overnight. After washing the plate, 100 µl of a 1,000-fold solution of a rabbit polyclonal antibody (OCT701) against TNF-α was added and incubated for 2 hours at room temperature. The plate was washed and 100 µl of (goat) POD-labeled anti-rabbit IgG was added to each well. After incubation for 2 hours at room temperature, the plate was washed and the enzymatic activity as OD₄₉₂ adsorbance was measured using o-phenylenedimine as a substrate.

The results are shown in Table 7.

**TABLE 7**

| | | | | | |
|---|---|---|---|---|---|
| TNF-α concentration (pg/ml) | 0 | 9.375 | 37.50 | 150.0 | 600.0 |
| Adsorbance 1 | 89 | 117 | 205 | 535 | 1510 |
| Adsorbance 2 | 95 | 123 | 206 | 540 | 1481 |
| Adsorbance 3 | 95 | 124 | 209 | 514 | 1487 |
| Mean | 93 | 121 | 207 | 530 | 1490 |
| Mean -Blank | 0 | 28 | 114 | 437 | 1397 |
| C. V. (%) | 3.7 | 3.1 | 1.0 | 2.6 | 0.7 |

## Claims

1. Monoclonal antibodies exhibiting specific reactivities to human TNF-α, obtainable from hybridoma KOCO 705 (FERM BP-2569).

## Patentansprüche

1. Aus Hybridom KOKO 705 (FERM BP-2569) erhältliche monoklonale Antikörper, die spezifische Reaktivitäten gegenüber menschlichem TNF-α zeigen.

## Revendications

1. Anticorps monoclonaux présentant des réactivités spécifiques avec TNF-α humain, pouvant être obtenus à partir de l'hybridome KOCO 705 (FERM BP-2569).
